# EUROPEAN PATENT APPLICATION

(11) **EP 4 534 699 A1**
(43) Date of publication of application: **09.04.2025**
(21) Application number: 23866697.8
(22) Date of filing: 30.10.2023
(51) Int. Cl.: C12Q 1/6886

(54) **CERVICAL CELL GENE METHYLATION DETECTION KIT AND DETECTION METHOD THEREFOR**

(30) Priority: 14.08.2023 CN 202311016237
(71) Applicant: Qingdao Ruiside Medical Laboratory Co., Ltd., Qingdao, Shandong 266000 (CN)
(72) Inventor: ZHANG, Bingqiang, Qingdao, Shandong 266000 (CN); CHEN, Mengmeng, Qingdao, Shandong 266000 (CN); JU, Junmei, Qingdao, Shandong 266000 (CN); ZHOU, Yang, Qingdao, Shandong 266000 (CN); SUN, Yundong, Qingdao, Shandong 266000 (CN); ZHAO, Yunxue, Qingdao, Shandong 266000 (CN); HAN, Lihui, Qingdao, Shandong 266000 (CN); LIU, Shili, Qingdao, Shandong 266000 (CN); LUAN, Yansong, Qingdao, Shandong 266000 (CN)
(74) Representative: Cleanthous, Marinos
(86) International application number: PCT/CN2023/127729
(87) International publication number: WO 2025/035597

(57) **Abstract**

The present invention pertains to the technical field of biomedical testing, and more specifically pertains to a kit and method for detecting methylation of genes in cervical cells. The present invention is used to determine whether a precancerous lesion occurs in a sample by detecting a methylation level of promoter regions of three genes MTHFR, PAX1 and SGSH in the sample, which is easy and convenient to operate, has high detection sensitivity and good specificity, and has a very positive significance for the detection of cervical cancer.

## Description

### TECHNICAL FIELD

The present invention pertains to the technical field of biomedical testing, and more specifically pertain to a kit and method for detecting methylation of genes in cervical cells based on fluorescence quantitative PCR technology.

### BACKGROUND

In China, cervical cancer is one of the common malignant tumors threatening women's health, with incidence and mortality ranking second. In 2018, there were more than 569,000 new cases of cervical cancer and more than 311,000 deaths worldwide, with 85% of these cases occurring in developing countries. About 111,000 new cases and 34,000 deaths occurred in China in 2015. In general, the distribution of deaths caused by cervical cancer in rural areas is slightly higher than that in urban areas, and that in central and western areas is about twice as much as that in eastern areas. The median age of onset of cervical cancer is 51 years old in China. However, it mainly occurs in two age groups, with the most being 40-50 years old and a peak appearing at 60-70 years old, and it is rarely seen before 20 years old. However, it is worth noting that in recent years, the average age of onset of cervical cancer is gradually decreasing, and there is a trend of younger cases. Therefore, it is necessary to standardize the diagnosis and treatment of cervical cancer nationwide. On the other hand, the occurrence of cervical cancer can be effectively controlled through examination and treatment of precancerous lesions.

At present, the cytologic TCT and virologic HPV tests currently used in clinical practice have limitations and cannot fully meet clinical needs. The methylation detection of cervical cancer genes has high sensitivity, specificity and accuracy, and can effectively fill in the current clinical gap and provide more valuable references for clinicians, thus better serving clinical practice and the public.

### SUMMARY

In order to solve the problems of the prior art, the object of the present invention is to provide a kit and method for detecting methylation of genes in cervical cells based on fluorescence quantitative PCR technology so as to realize joint detection of multi-gene methylation and thus realize early screening of cervical cancer.

In the present invention, the fluorescence quantitative PCR technology is used to jointly detect methylation of MTHFR, PAX1 and SGSH genes in cervical exfoliated cells of patients. After samples are treated with bisulfite, specific primers and fluorescent probes are used to perform nucleic acid amplification to achieve rapid detection of methylation of methylation-related genes MTHFR, PAX1 and SGSH and provide a simple and accurate screening for clinical practice. The NCBI database number of the MTHFR gene sequence involved in the present invention is NC_000001.11, and the NCBI database numbers of the PAX1 gene and the SGSH gene are NC_000020.11 and NC_000017.11 respectively.

**In order to achieve the object of the present invention, a first technical solution provided by the present invention is as follows:** the present invention provides a kit for detecting gene methylation of cervical cells, wherein the kit comprises: an MTHFR PCR reaction solution for detecting a degree of methylation in at least one target region in a promoter region of an MTHFR gene; a PAX1 PCR reaction solution for detecting a degree of methylation in at least one target region in a promoter region of a PAX1 gene; an SGSH PCR reaction solution for detecting a degree of methylation in at least one target region in a promoter region of an SGSH gene; and a positive control and a negative control, wherein the promoter regions are not strictly defined as regions located within promoter fragments, but are gene fragments of regions in the vicinity of promoters.

The MTHFR gene, the PAX1 gene and the SGSH genes are all closely related to the progression of cervical cancer. A methylation detection region of each target gene contains multiple CpGs, which reduces randomness of base changes compared with the detection of a single base, and simultaneous joint methylation detection of the three target genes MTHFR, PAX1 and SGSH effectively makes up the differences which may be caused by site selection and reaction system, and improves the accuracy of detection of gene methylation in cervical cells.

The MTHFR PCR reaction solution comprises an MTHFR forward primer and an MTHFR reverse primer, an MTHFR probe, an internal reference ACTB forward primer and an internal reference ACTB reverse primer, and an internal reference ACTB probe; the PAX1 PCR reaction solution comprises a PAX1 forward primer and a PAX1 reverse primer, a PAX1 probe, an internal reference ACTB forward primer and an internal reference ACTB reverse primer, and an internal reference ACTB probe; and the SGSH PCR reaction solution comprises an SGSH forward primer and an SGSH reverse primer, an SGSH probe, an internal reference ACTB forward primer and an internal reference ACTB reverse primer, and an internal reference ACTB probe.

The MTHFR reaction solution, the PAX1 reaction solution and the SGSH PCR reaction solution each further comprise a 10× buffer, a 10mM dNTP mix and an enzyme mix. The enzyme mix comprises a Taq enzyme and an UNG enzyme, the Taq enzyme is a hot-start Taq enzyme, and the UNG enzyme is an uracil-N-glycosylase.

A base sequence of the MTHFR forward primer is tcggtttttattggtcgcgg (SEQ ID No. 1), a base sequence of the THFR reverse primer is tacctcgaaacaaaacgc (SEQ ID No. 2), and a base sequence of the MTHFR probe is accgcgaaaccaac (SEQ ID No. 3); a base sequence of the PAX1 forward primer istcgacgttgtagtttttcggttagacgaat (SEQ ID No. 4), a base sequence of the PAX1 reverse primer is tacccctccaaaacctcccac (SEQ ID No. 5), and a base sequence of the PAX1 probe is cccgaccccaacccaaataacttcat (SEQ ID No. 6); and a base sequence of the SGSH forward primer is gtttcgcgtcgcgttt (SEQ ID No. 7), a base sequence of the SGSH reverse primer is acgacgacgaaccacc (SEQ ID No. 8), and a base sequence of the SGSH probe is cgcgccctaaact (SEQ ID No. 9); and a base sequence of the internal reference ACTB forward primer is taggatttttatttag (SEQ ID No. 10), a base sequence of the internal reference ACTB reverse primer is tgtgaatttttgttat (SEQ ID No. 11), and a base sequence of the internal reference ACTB probe is ttttaagggaggagt (SEQ ID No. 12).

The MTHFR probe, the PAX1 probe, the SGSH probe and the ACTB probe are respectively labeled with a fluorescent reporter and a fluorescent quencher at both ends.

The fluorescent reporter is selected from a group consisting of FAM, HEX, ROX, JOE, VIC, TET, NED, FITC, CY3 or CY5; and the fluorescent quencher is selected from a group consisting of BHQ1, BHQ2, BHQ3, TAMRA, Eclipse and DABCYL.

Each of the probes are respectively labeled with a fluorescent reporter/a fluorescent quencher at both ends, and the fluorescent quenchers at 5 ends are identical or different and selected from a group consisting of FAM, HEX, ROX, JOE, VIC, TET, NED, FITC, CY3 or CY5; and the fluorescent quenchers at 3' ends are identical or different and selected from a group consisting of BHQ1, BHQ2, BHQ3, TAMRA, Eclipse and DABCYL.

In a specific embodiment of the present invention, the MTHFR fluorescent probe has a reporter FAM at the 5' end and a quencher BHQ1 at the 3' end, the internal reference gene fluorescent probe has a fluorescent reporter VIC at the 5' end and a quencher BHQ1 at the 3' end; the PAX1 fluorescent probe has a reporter FAM at the 5' end and a quencher BHQ1 at the 3' end; the internal reference gene fluorescent probe has a fluorescent reporter VIC at the 5' end and a quencher BHQ1 at the 3' end; the SGSH fluorescent probe has a luminophore FAM at the 5' end and a quencher BHQ1 at the 3' end; and the internal reference gene fluorescent probe has a fluorescent reporter VIC at the 5' end and a quencher BHQ1 at the 3' end.

The positive control is selected from a DNA of a cervical squamous cell cancer cell Hisa; and
the negative control is sterile water.

**In order to achieve the object of the present invention, a second technical solution provided by the present invention is as follows:** the present invention provides a method for detecting methylation of genes in cervical cells, which comprises the following steps:
(1) extracting a DNA from a sample to be detected;
(2) transforming the DNA of the sample to be detected with bisulfite to obtain purified Bis-DNA;
(3) performing a PCR amplification reaction using the purified Bis-DNA as a template using a combination of the primers and the probes in the MTHFR PCR reaction solution, the PAX1 PCR reaction solution and the SGSH PCR reaction solution in the kit according to the present invention: selecting ACTB as an internal reference gene for the three target genes namely the MTHFR gene, the PAX1 gene and the SGSH gene, and achieving simultaneous detection of the target genes and the internal reference gene according to multi-channel fluorescent probe labeling; and
(4) obtaining △Ct values of the three target genes namely the MTHFR gene, the PAX1 gene and the SGSH gene according to the above steps (a target Ct value - an internal reference Ct value), that is, X1=Ct of MTHFR - Ct of ACTB, X2 = Ct of PAX1 - Ct of ACTB, X3 = Ct of SGSH - Ct of ACTB, substituting the △ Ct values into a regression equation Y=10.326-0.309*X1-0.623*X2-0.552*X3 to determine the degree of methylation; determining the sample to be detected to be positive for cervical cancer methylation when Y ≥ 0; and determining the sample to be detected to be negative for cervical cancer methylation when Y < 0.

The present invention achieves the following advantages:
1. The present invention provides a combination of methylation genes for methylation detection of cervical cancer, comprising the following methylation genes MTHFR, PAX1 and SGSH. In the present invention, the MTHFR gene, the PAX1 gene and the SGSH gene are all closely related to the progression of cervical cancer. The methylation detection region of each target gene contains multiple CpGs, which reduces the randomness of base changes compared with the detection of a single base. The determination of methylation of the target genes is not limited to one or two bases.
2. ACTB is selected as the internal reference gene for the three target genes. The target genes and the internal reference genes are simultaneously detected according to the multi-channel fluorescent probe labeling. Finally, the △Ct values of the three genes (target Ct value- internal reference Ct value) are substituted into the regression equation Y=10.326-0.309*X1-0.623*X2-0.552*X3 to determine the degree of methylation.
3. The present invention provides a reaction solution and detection method for methylation PCR of cervical cancer, and provides a matching method for nucleic acid extraction from a sample and sulfite transformation, wherein the sulfite-transformed DNA is used as a template for the PCR reaction while achieving the detection of the three target genes MTHFR, PAX1 and SGSH. By combining the MTHFR, PAX1 and SGSH methylation detection, the present invention effectively makes up the differences which may be caused by site selection and reaction system, and improves the accuracy of methylation detection for cervical cancer.

### BRIEF DESCRIPTION OF THE DRAWINGS

FIG. 1 shows typical methylation PCR amplification curves of genes (MTHFR, PAX1 and SGSH) in cervical cancer exfoliated cell samples, (a kit with primer and probe combinations of MTHFR(F/R/P), PAX1(F/R/P) and SGSH(F/R/P)) indicating that Ct values of the MTHFR gene, the PAX1 gene and the SGSH gene in the cervical cancer exfoliated cell samples are ≤ 32;
FIG. 2 shows typical non-methylated PCR amplification curves of genes (MTHFR, PAX1 and SGSH) in normal cervical exfoliated cell samples, (a kit with primer and probe combinations of MTHFR(F/R/P), PAX1(F/R/P) and SGSH(F/R/P)) indicating that the Ct values of the MTHFR gene, the PAX1 gene and the SGSH gene in the normal cervical exfoliated cell samples are > 32;
FIG. 3 shows a comparison of cervical precancerous lesions (CIN2 and above) and normal controls, wherein area under the ROC curve (AUC) is 0.965 (95%CI=0.914-1; P < 0.0001);
FIG. 4 is a table of parameter setting for fluorescence detection channel selection and amplification cycle according to Example 2 of the present invention; and
FIG. 5 is a table of comparison between detection results of a kit according to Example 3 of the present invention and pathological detection results.

### DESCRIPTION OF THE EMBODIMENTS

### (I) Example 1: A kit for detecting methylation of genes in cervical cells according to the present invention

A kit for detecting methylation of genes in cervical cells according to the present invention comprises: an MTHFR PCR reaction solution for detecting a degree of methylation in at least one target region in a promoter region of an MTHFR gene; a PAX1 PCR reaction solution for detecting a degree of methylation in at least one target region in a promoter region of a PAX1 gene; an SGSH PCR reaction solution for detecting a degree of methylation in at least one target region in a promoter region of an SGSH gene, and a positive control and a negative control; wherein the promoter regions were not strictly defined as regions located within promoter fragments, but were gene fragments of regions in the vicinity of promoters.

The MTHFR PCR reaction solution comprised an MTHFR forward primer, an MTHFR reverse primer, an MTHFR probe, an internal reference ACTB forward primer, an internal reference ACTB reverse primer, an internal reference ACTB probe, a 10× buffer, a 10mM dNTP mix and an enzyme mix.

The PAX1 PCR reaction solution comprised a PAX1 forward primer, a PAX1 reverse primer, a PAX1 probe, an internal reference ACTB forward primer, an internal reference ACTB reverse primer, an internal reference ACTB probe, a 10× buffer, a 10mM dNTP mix and an enzyme mix.

The SGSH PCR reaction solution comprised an SGSH forward primer, an SGSH reverse primer, an SGSH probe, an internal reference ACTB forward primer, an internal reference ACTB reverse primer, an internal reference ACTB probe, a 10× buffer, a 10mM dNTP mix and an enzyme mix.

The ratio of the forward primer, the reverse primer and the probe in the MTHFR PCR reaction solution, the PAX1 PCR reaction solution and the SGSH PCR reaction solution described above was 4:4:2; and the forward primer was 200nM, the reverse primer was 200nM, and the probe was 100nM.

The enzyme mix comprised a Taq enzyme and an UNG enzyme, the Taq enzyme was a hot-start Taq enzyme, and the UNG enzyme was an uracil-N-glycosylase.

Nucleotide sequences of the cervical cancer methylation genes MTHFR, PAX1 and SGSH primer probes and the internal reference gene ACTB primer probes involved in this Example can be found in Table 1 below. The nucleotide sequence tables related to the present invention have been submitted to China National Intellectual Property Administration, and they complied with **standard ST.26.**

**Table 1**

| Sequence name | Oligonucleotide sequence 5'-3' | No. |
|---|---|---|
| MTHFR forward primer | tcgetttttattggtcgcge | SEQ ID No. 1 |
| MTHFR reverse primer | tacctcgaaacaaaacgc | SEQ ID No. 2 |
| MTHFR probe | accgcgaaaccaac | SEQ ID No. 3 |
| PAX1 forward primer | tcgacgttgtagtttttcggttagacgaat | SEQ ID No. 4 |
| PAX1 reverse primer | tacccctccaaaacctcccac | SEQ ID No. 5 |
| PAX1 probe | cccgaccccaacccaaataacttcat | SEQ ID No. 6 |
| SGSH forward primer | gtttcgcgtcgcgttt | SEQ ID No. 7 |
| SGSH reverse primer | acgacgacgaaccacc | SEQ ID No. 8 |
| SGSH probe | cgcgccctaaact | SEQ ID No. 9 |
| Internal reference ACTB forward primer | taggatttttatttag | SEQ ID No. 10 |
| Internal reference ACTB reverse primer | tgtgaatttttgttat | SEQ ID No. 11 |
| Internal reference ACTB probe | ttttaagggaggagt | SEQ ID No. 12 |

Further, the positive control was selected from a DNA of a cervical squamous cell cancer cell Hisa.

Further, the negative control was sterile water.

The specific components of the kit are shown in the table below:

| Component | Main components | Specification |
|---|---|---|
| MTHFR PCR reaction solution | MTHFR forward primer, MTHFR reverse primer, MTHFR probe, internal reference ACTB forward primer, internal reference ACTB reverse primer, internal reference ACTB probe, 10× buffer, 10mM dNTP mix, Taq enzyme and UNG enzyme. | 400ul |
| PAX1 PCR reaction solution | PAX1 forward primer, PAX1 reverse primer, PAX1 probe, internal reference ACTB forward primer, internal reference ACTB reverse primer, internal reference ACTB probe, 10× buffer, 10mM dNTP mix, Taq enzyme and UNG enzyme. | 400ul |
| SGSH PCR reaction solution | SGSH forward primer, SGSH reverse primer, SGSH probe, internal reference ACTB forward primer, internal reference ACTB reverse primer, internal reference ACTB probe, 10× buffer, 10mM dNTP mix, Taq enzyme and UNG enzyme. | 400ul |
| Positive control | Hisa cell DNA | 50ul |
| Negative control | Sterile water | 50ul |

### (II) Example 2

A method for detecting methylation of genes in cervical cells using the kit for detecting methylation of genes in cervical cells according to **the present invention** comprised the following steps:
(1) A DNA was extracted from a sample to be detected.
   At this step, a cell DNA was extracted from a cervical exfoliated cell sample. After extraction, a concentration of the DNA and OD260/OD280 were measured using a Nano-500 micro-spectrophotometer, with the OD260/OD280 ranging from 1.6 to 2.0.
(2) The DNA of the sample to be detected was transformed with bisulfite to obtain purified Bis-DNA.

At this step, after the extraction of the cell DNA, the extracted DNA was transformed with bisulfite, with unmethylated cytosine c transformed into uracil (U) and methylated cytosine (C) remaining unchanged, resulting in the purified Bis-DNA. The obtained Bis-DNA should be detected in a timely manner or stored at - 20°C for not more than 4 months for detection, or stored at - 80°C for long-term storage.
(3) Using the purified Bis-DNA as a template, a PCR amplification reaction was performed using a combination of the primers and the probes in the MTHFR PCR reaction solution, the PAX1 PCR reaction solution and the SGSH PCR reaction solution in the kit according to the present invention: ACTB was selected as an internal reference gene for the three target genes namely the MTHFR gene, the PAX1 gene and the SGSH gene, and simultaneous detection of the target genes and the internal reference gene was achieved according to multi-channel fluorescent probe labeling. The specific operations are as follows:

### S1. Reagent preparation

An MTHFR gene PCR reaction system was composed of the following reagent components: the MTHFR PCR reaction solution had a volume of 15 uL, the Bis-DNA template had a volume of 2-5 uL, and purified water had a volume up to 20 uL.

A PAX11 gene PCR reaction system was composed of the following reagent components: the PAX1 PCR reaction solution had a volume of 15 uL, the Bis-DNA template had a volume of 2-5 uL, and purified water had a volume up to 20 uL.

An SGSH gene PCR reaction system was composed of the following reagent components: the SGSH PCR reaction solution had a volume of 15 uL, the Bis-DNA template had a volume of 2-5 uL, and purified water had a volume up to 20 uL.

### S2. Sample adding

A total of 2-5 µL of the nucleic acid sample to be detected was added to eight-row tubes, with a final volume of 20 µL per tube. Each tube was tightly stoppered, and centrifuged at a low speed instantaneously for detection on a PCR amplifier.

### S3. PCR amplification

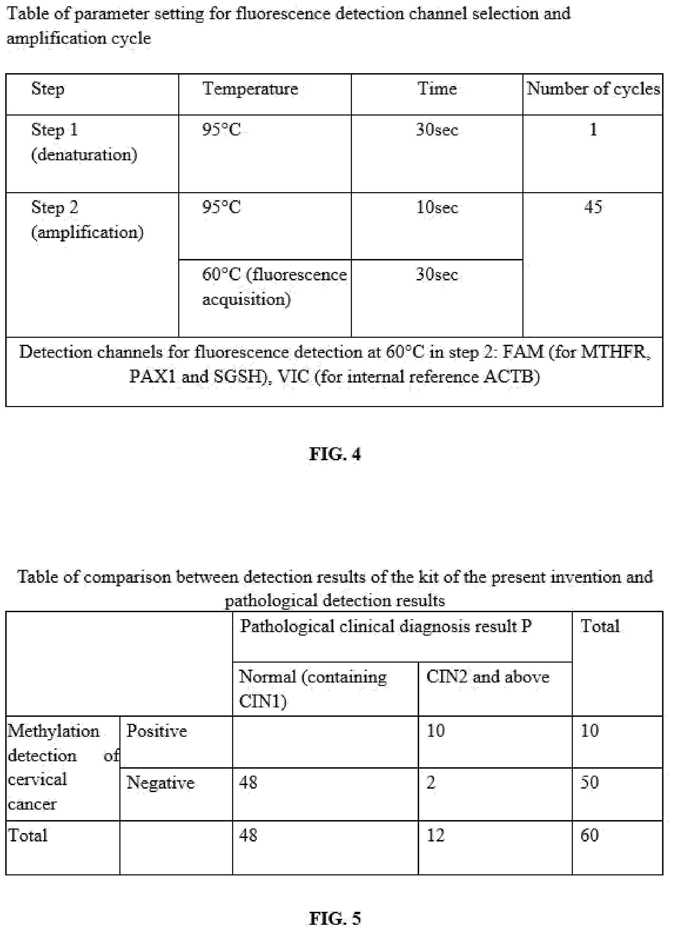

Fluorescence detection channels were selected and amplification cycle parameters were set as shown in FIG. 4. Note: ROX correction was not selected, and None was selected for quenchers. After setup, a corresponding file was saved, and a reaction program was run.

(4) △Ct values of the three target genes namely the MTHFR gene, the PAX1 gene and the SGSH gene were obtained according to the above steps (a target Ct value - an internal reference Ct value), that is, X1 = Ct of MTHFR - Ct of ACTB, X2 = Ct of PAX1 - Ct of ACTB, X3 = Ct of SGSH - Ct of ACTB, the △Ct values were substituted into a regression equation Y=10.326-0.309*X1-0.623*X2-0.552*X3 to determine the degree of methylation; the sample to be detected was determined to be positive for cervical cancer methylation when Y ≥ 0; and the sample to be detected was determined to be negative for cervical cancer methylation when Y < 0.

### (III) Example 3: Detection accuracy test of the kit

A comparison between detection results of the kit according to the present invention and pathological detection results is as shown in FIG. 5.

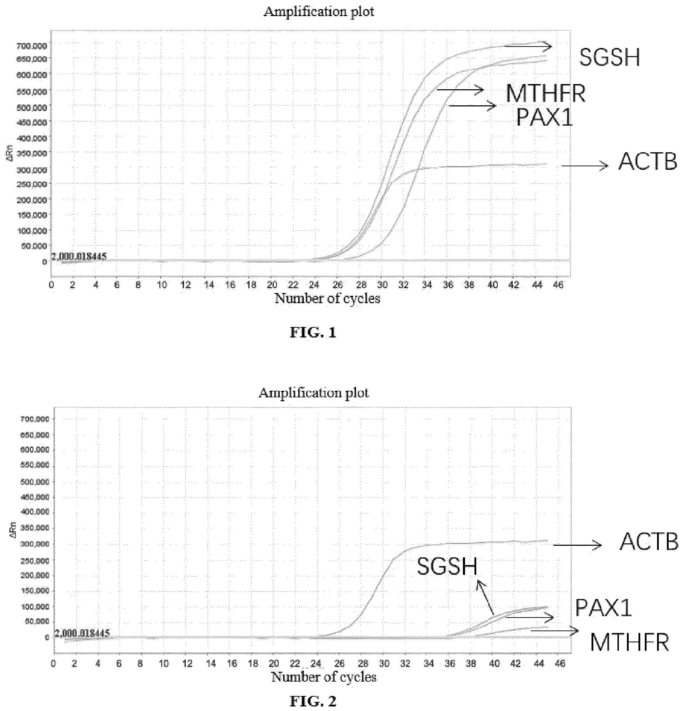

Methylation detection results of genes (MTHFR, PAX1 and SGSH) in 60 cervical exfoliated cell samples are as shown in FIGS. 1 and 2. FIG. 1 shows typical methylation PCR amplification curves of the genes (MTHFR, PAX1 and SGSH) in the cervical cancer exfoliated cell samples, (a kit with primer and probe combinations of MTHFR(F/R/P), PAX1(F/R/P) and SGSH(F/R/P)) indicating that Ct values of the MTHFR gene, the PAX1 gene and the SGSH gene in the cervical cancer exfoliated cell samples were ≤ 32. FIG. 2 shows typical non-methylated PCR amplification curves of genes (MTHFR, PAX1 and SGSH) in normal cervical exfoliated cell samples, (a kit with primer and probe combinations of MTHFR(F/R/P), PAX1(F/R/P) and SGSH(F/R/P)) indicating that the Ct values of the MTHFR gene, the PAX1 gene and the SGSH gene in the normal cervical exfoliated cell samples are > 32. The experimental data showed that using CIN2 as a cut-off value for clinical diagnosis, the detection sensitivity of the kit provided in Example 1 of the present invention was 83.3% and 100% respectively.

### (IV) Example 4: Pathological diagnosis results: ROC curve of normal individuals and CIN2 and above

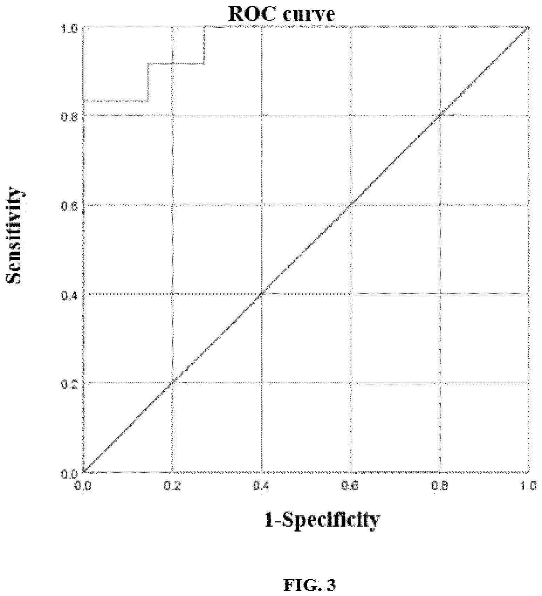

As shown in FIG. 3, the kit provided in Example 1 of the present invention can clearly distinguish cervical precancerous lesions (CIN2 and above) from normal controls, and the AUC of the ROC curve was 0.965 (95% CI=0.914-1; P < 0.0001).

### (V) Example 5: Logistic regression analysis

A logistic regression equation for determining normal samples and CIN2 and above was obtained through experiments: Y *X1+ A2*X2+A3*X3; where A0, A1, A2 and A3 were clinical coefficients, and a weight relationship of the clinical coefficients was as follows: MTHFR proportion coefficient A1=-0.309, PAX1 proportion coefficient A2 =-0.623, SGSH proportion coefficient A3 =-0.552, and constant proportion coefficient A0 = 10.326.

Differences in amplification cycles of the MTHFR, PAX1, SGSH genes and the internal reference gene ACTB were X1, X2, and X3 respectively. X1 = Ct of MTHFR - Ct of ACTB, X2 = Ct of PAX1 - Ct of ACTB and X3 = Ct of SGSH - Ct of ACTB were substituted into a regression equation Y=10.326-0.309*X1-0.623*X2-0.552*X3 to determine the degree of methylation; the sample to be detected was determined to be positive for cervical cancer methylation when Y ≥ 0; and the sample to be detected was determined to be negative for cervical cancer methylation when Y < 0.

The sensitivity and specificity of joint detection of cervical cancer and precancerous lesions by methylation fitting regression analysis of the three genes were much higher than those of individual detection analysis results of the MTHFR, PAX1 and SGSH genes.

## Claims

1. A kit for detecting methylation of genes in cervical cells, comprising: an MTHFR PCR reaction solution for detecting a degree of methylation in at least one target region in a promoter region of an MTHFR gene; a PAX1 PCR reaction solution for detecting a degree of methylation in at least one target region in a promoter region of a PAX1 gene; an SGSH PCR reaction solution for detecting a degree of methylation in at least one target region in a promoter region of an SGSH gene; and a positive control and a negative control, wherein the promoter regions are not strictly defined as regions located within promoter fragments, but are gene fragments of regions in the vicinity of promoters.

2. The kit according to claim 1, wherein:
the MTHFR PCR reaction solution comprises an MTHFR forward primer and an MTHFR reverse primer, an MTHFR probe, an internal reference ACTB forward primer and an internal reference ACTB reverse primer, and an internal reference ACTB probe; the PAX1 PCR reaction solution comprises a PAX1 forward primer and a PAX1 reverse primer, a PAX1 probe, an internal reference ACTB forward primer and an internal reference ACTB reverse primer, and an internal reference ACTB probe; and the SGSH PCR reaction solution comprises an SGSH forward primer and an SGSH reverse primer, an SGSH probe, an internal reference ACTB forward primer and an internal reference ACTB reverse primer, and an internal reference ACTB probe.

3. The kit according to claim 2, wherein the MTHFR reaction solution, the PAX1 reaction solution and the SGSH PCR reaction solution each further comprise a 10× buffer, a 10mM dNTP mix and an enzyme mix, the enzyme mix comprises a Taq enzyme and an UNG enzyme, the Taq enzyme is a hot-start Taq enzyme, and the UNG enzyme is an uracil-N-glycosylase.

4. The kit according to claim 2, wherein a base sequence of the MTHFR forward primer is tcggtttttattggtcgcgg (SEQ ID No. 1), a base sequence of the THFR reverse primer is tacctcgaaacaaaacgc (SEQ ID No. 2), and a base sequence of the MTHFR probe is accgcgaaaccaac (SEQ ID No. 3); a base sequence of the PAX1 forward primer istcgacgttgtagtttttcggttagacgaat (SEQ ID No. 4), a base sequence of the PAX1 reverse primer is tacccctccaaaacctcccac (SEQ ID No. 5), and a base sequence of the PAX1 probe is cccgaccccaacccaaataacttcat (SEQ ID No. 6); and a base sequence of the SGSH forward primer is gtttcgcgtcgcgttt (SEQ ID No. 7), a base sequence of the SGSH reverse primer is acgacgacgaaccacc (SEQ ID No. 8), and a base sequence of the SGSH probe is cgcgccctaaact (SEQ ID No. 9); and a base sequence of the internal reference ACTB forward primer is taggatttttatttag (SEQ ID No. 10), a base sequence of the internal reference ACTB reverse primer is tgtgaatttttgttat (SEQ ID No. 11), and a base sequence of the internal reference ACTB probe is ttttaagggaggagt (SEQ ID No. 12).

5. The kit according to claim 2, wherein the MTHFR probe, the PAX1 probe, the SGSH probe and the ACTB probe are respectively labeled with a fluorescent reporter and a fluorescent quencher at both ends; the fluorescent reporter is selected from a group consisting of FAM, HEX, ROX, JOE, VIC, TET, NED, FITC, CY3 or CY5; and the fluorescent quencher is selected from a group consisting of BHQ1, BHQ2, BHQ3, TAMRA, Eclipse and DABCYL.

6. The kit according to claim 1, wherein the positive control is selected from a DNA of a cervical squamous cell cancer cell Hisa; and the negative control is sterile water.

7. A method for detecting methylation of genes in cervical cells, comprising the following steps:
(1) extracting a DNA from a sample to be detected;
(2) transforming the DNA of the sample to be detected with bisulfite to obtain purified Bis-DNA;
(3) performing a PCR amplification reaction using the purified Bis-DNA as a template using a combination of the primers and the probes in the MTHFR PCR reaction solution, the PAX1 PCR reaction solution and the SGSH PCR reaction solution in the kit according to claim 1: selecting ACTB as an internal reference gene for the three target genes namely the MTHFR gene, the PAX1 gene and the SGSH gene, and achieving simultaneous detection of the target genes and the internal reference gene according to multi-channel fluorescent probe labeling; and
(4) obtaining △Ct values of the three target genes namely the MTHFR gene, the PAX1 gene and the SGSH gene according to the above steps (a target Ct value - an internal reference Ct value), that is, X1 = Ct of MTHFR - Ct of ACTB, X2 = Ct of PAX1 - Ct of ACTB, X3 = Ct of SGSH - Ct of ACTB, substituting the △ Ct values into a regression equation Y=10.326-0.309*X1-0.623*X2-0.552*X3 to determine the degree of methylation; determining the sample to be detected to be positive for cervical cancer methylation when Y ≥ 0; and determining the sample to be detected to be negative for cervical cancer methylation when Y < 0.
